Europäisches Patentamt

European Patent Office

Office européen des brevets

(11) Publication number: **0 099 133**
**B1**

## EUROPEAN PATENT SPECIFICATION

(45) Date of publication of patent specification: **23.11.88**

(51) Int. Cl.⁴: **A 61 K 39/395, C 07 K 15/06**

(21) Application number: **83106965.3**

(22) Date of filing: **15.07.83**

(54) **Anti-cancer drugs comprising platinum complexes.**

(30) Priority: **15.07.82 IL 66338**

(43) Date of publication of application:
**25.01.84 Bulletin 84/04**

(45) Publication of the grant of the patent:
**23.11.88 Bulletin 88/47**

(84) Designated Contracting States:
**AT BE CH DE FR GB IT LI LU NL SE**

(56) References cited:
CHEMICAL ABSTRACTS, vol. 98, no. 14, 4th
April 1983, page 377, no. 113588q, Columbus,
Ohio, US, E.HURWITZ :"Attempts at site-
directed experimental chemotherapy with
antibody drug-conjugates"

CHEMICAL ABSTRACTS, vol. 97, no. 19, 8th
November 1982, page 46, no. 156098b,
Columbus, Ohio, US E.Hurwitz et
al:"Plantanium-complexed antitumor
imsynthesis of mouse tumor cells"

(73) Proprietor: **YEDA RESEARCH AND
DEVELOPMENT COMPANY, LTD.**
**Weizmann Institute of Science Herzl Street at
Yavne Road P.O. Box 95
Rehovot 76100 (IL)**

(72) Inventor: **Arnon, Ruth**
**Meonot Wix Weizmann Inst. of Science P.O. Box
26
Rehovot, 76 100 (IL)**
Inventor: **Hurwitz, Esther**
**Rhov Hess 18
Rehovot (IL)**
Inventor: **Kashi, Rena**
**Mivza Kadesh, 7
Rishon Le Zion (IL)**
Inventor: **Wilchek, Meir**
**Rh. Havoda 3
Rehovot (IL)**
Inventor: **Sela, Michael**
**Neve Weizmann, W.I.S.
Rehovot, 76.100 (IL)**
Inventor: **Schechter, Bilha**
**Meonot Brazil 1, W.I.S.
Rehovot, 76.100 (IL)**

Courier Press, Leamington Spa, England.

EP 0 099 133 B1

(56) References cited:

NATO ADV. STUDY INST. SER., SER. A, vol. 47, 1982, pages 31-54, Targeting Drugs, R.ARNON: "Antibodies and dextran as anti-tumour drug carriers"p.38, lines 14-28

CHEMICAL ABSTRACTS, vol. 98, no. 14, 4th April 1983, page 380, no. 113624y, Columbus, Ohio, US E.Hurwitz:"Specific and nonspecific macromolecule-drug conjugates for the improvment of cancer chemotherapy".

(74) Representative: **Patentanwälte Zellentin & Partner**
**Zweibrückenstrasse 15**
**D-8000 München 2 (DE)**

## Description

### Field of the invention

The present invention relates to novel anti-cancer drugs. According to the invention there are provided platinum complexed anti-tumor immunoglobulins (Igs) which are effective anti-tumor agents which retain their specific anti-body binding capacity. The invention further relates to the preparation of the novel drugs defined above. The binding is effected via dextran-amine moieties.

### Background of the invention

Platinum containing complexes in general and Cis-diamino-platinum dihalides (Cis-DDP) in particular are effective anti-tumor agents (Rosenberg et al, Nature (1969), 222, 385-6; Leh, J. Pharm. Sci. (1976), 65, 315. The therapeutic effectivity of such complexes was demonstrated in various experimental animal models (Rosenberg et al, Cancer Res (1970), 30, 1799 and others). Furthermore, Cis-DDP is now widely used clinically and is an effective drug against metastatic testicular and ovarian carcinoma.

The major drawback of Cis-DDP, as of other anti-cancer agents, is its high degree of toxicity. Other platinum complexes such as condensation products with various amides, mainly the nucleic acid bases uracil and thymine (platinum blues) or platinum complexes with diaminocyclohexanes have been developed. Many such diamino-cis-platinum derivates exhibit anti-tumor activity in vivo and are less toxic.

Anti-tumor antibodies have been used as carriers of a number of chemotherapeutic agents in an attempt to increase their specificity towards tumor cells and thereby reduce their general toxicity (Hurwitz et al, Cancer res. (1975), 35, 1175). It was also shown that mixtures of anti-tumorantibodies and drugs were more effective than either of them separately, when applied in vivo in experimental tumor systems. See also U.S. Patents Nos. 4,263,279, and 4,093,607.

The direct bonding of platinum to antibodies and the use of such complexes against tumor systems is mentioned in Hurwitz et al., Benzon Symp. 1981, published 1982 and Hurwitz et al., I. Natl. Canc. Institut 1982, 47—51.

### Summary of the invention

According to the present invention there are provided novel platinum salts complexed via a dextran-amine moiety to anti-tumor immunoglobulins which are useful as drugs for the treatment of certain tumors. The novel pharmaceutically active complexes are prepared by complexing platinum salts with anit-tumor reactive immunoglobulins coupled to a dextran-amine moiety. When said Ig, which has a binding affinity towards certain tumor cells, is complexed with certain platinum salts, highly effective inhibitors of DNA synthesis are obtained. Such complexes are much more effective in inhibiting DNA synthesis than those obtained with normal Ig or with serum albumin. Especially effective preparations are obtained when platinum salts like $K_2PtCl_4$ are complexed.

The complexes are prepared by reacting said immunoglobulin (Ig) with the desired platinum salt in a suitable medium, such as phosphate buffered saline, and separating the unbound platinum from the product.

The toxicity of the novel complexes is lower than that of the conventional cis-diamino-platinum hydrochloride. When equal quantities are administered, the anti-tumor efficacy of the novel complexes is similar to that of the conventional platinum compound.

The invention is illustrated with reference to the following illustrative examples, which are to be construed in a nonlimitative manner. The resulting material was applied to a Biogel P-60 column.

### Example

Platinum-dextran amine-Ig complex.

### Tumor cells

Three types of tumor cells were used: (a) Moloney virus-induced lymphoma, YAC (Klein E., Klein G., J. Nat Cancer Inst, (1964), 32, 547—568) maintained in vivo in A/J male mice, and (b) a B cell leukemia, 38c 13 produced in C3H/eB mice by induction with 7,12-dimethylbenz (a) anthracene (Haran-Chera N., Peled A., Nature (1971), 24, 396—398). For the experiments the tumor cells were kept in culture in RPMI-1640 media containing 10% fetal calf serum (FCS), glutamine antibiotics and $15^{-5}M$ β-mercaptoethanol in the case of 38c 13 ells (Bergman Y., Haimovich J., Eur. J. Immunol., (1977), 7, 413—417) and (c) the metastatic Lewis lung carcinoma (3LL), a spontaneous tumor originated in 1951 in the lung of a C57BL/6 mouse (Sugiura, K., Stock, C.C. Cancer Res. (1955), 15, 38—51), maintained in C57BL/6 male mice, or in culture in RPMI-1640 medium containing 10% horse serum, glutamine and antibiotics.

### Immunoglobulins

Anti-YAC was produced in goats by injection of a soluble papain digest of the YAC cell-membrane as described previously (Hurwitz E. et al, Int. J. Cancer, (1978), 21, 747—755). The anti-38c 13 was produced by injecting goats with a 38c 13 cell-surface IgM. In each case the Ig fraction was precipitated from the antisera by ammonium sulfate at 33% saturation. A mouse anti-YAC IgM was produced from a hybridoma line according to Köhler G., Milstein C., J. Immunol., (1976), 6, 511—519. The IgM was precipitated from mouse ascitic fluid by ammonium sulfate at 45% saturation. Rat anti-3LL Ig's were produced from hyrbidoma lines. The Ig was precipitated from mouse ascitic fluid by ammonium sulfate at 45% saturation.

Platinum-Dex $NH_2$-Ig complex preparations—Dextran (MW 10.000) was oxidized with a 40 fold molar excess of sodium periodate in double distilled water (DDW) for 16 h. (2,5 gr dextran in 100 ml DDW). The oxidized dextran was then dialyzed against DDW, lyophilized, and reacted with a 130 molar excess of ethylenediamine in DDW at pH 8,6 for 6 hours. The Schiff bases formed between aldehyde groups on the oxidized dextran and the ethylenediamine were reduced with an equimolar amount of sodium cyanoborohydride at pH 7,5 for 3 hrs, the product was dialyzed against DDW and lyophilized. The product (Dex-NH$_2$) formed which contained

$$4—8—NH—CH_2—CH_2—NH_2$$

groups and another

$$8—12—NH—CH_2—CH_2NH—$$

groups per dextran molecule, is capable of binding 15—20 platinum molecules when interacted with K$_2$PtCl$_4$. Dex-NH$_2$, at a molar excess of 20:1 was coupled via its free NH$_2$ groups to Ig using a 300 molar excess of the bifunctional coupling reagent ethyl-3-(3-dimethyl-aminopropyl) carbodiimide hydrochloride (w/s DCC). Ig and Dex-NH$_2$ in PBS at pH5 were mixed with w/s DCC for 5 min, and the pH was then elevated to 7,8.

The reaction mixture was incubated at 4°C for 5 hours. The Dex-NH$_2$-Ig was precipitated twice with 75% ammonium sulfate to remove unbound Dex-NH$_2$, and dialyzed against PBS. The Dex-NH$_2$-Ig contained 2,5—3 molecules of Dex-NH$_2$ per Ig molecule. Platinum-Dex-NH$_2$-Ig complexes were prepared by reacting Dex-NH$_2$-Ig (final concentration 10—15 mg/ml 0,4M NaCl in PBS) with a 200 molar excess of K$_2$PtCl$_4$ (final concentration 5—8 mg/ml) for 16 hrs at 4°C. No precipitation of Ig occured. The resulting complex was either applied to Biogel P-60 column run in 0,1 M phosphate buffer at pH 6,4 for the separation of bound from free platinum or dialysed against PBS, to remove unbound K$_2$PtCl$_4$. The amount of complexed platinum was assayed (a) by a combination of ion exchange, resin loaded papers and X-ray spectrography (Campbell W. J. et al. Anal Chem 1966, 38, 987—996) and (b) the unbound platinum was determined spectrophoto-metrically (Golla Ed et al, Talanta (1972), 20, 199—210), the complexed platinum was obtained from the difference between the total and the unbound. Free Pt was separated from bound by ultrafiltration through conical membranes (Centriflo, Amicon, Holland).

Evaluation of anti-tumor activity

Inhibition of DNA synthesis in tumor cells by novel platinum -Dex-NH$_2$-Ig complexes was determined by a method similar to the one described previously by Hurwitz et al, Cancer Res., (1975), 35, 1175. The tumor cells in numbers ranging from 5×10$^4$—5×10$^5$/ml were suspended in their suitable growth media (RPMI-1640 containing 10% fetal calf serum, (FCS). The cells were dispensed in 100 l aliquots into the wells of microliter plates (Falcon, Microtest II 3040). The platinum compounds were added to the cells in 50 μl

amounts. The plates were incubated for 2—3 hours at 37°C in a humidified atmosphere of 5% CO$_2$ in air. At that time, 10 μl containing 0,5 μCi[$^3$H]-methylmidine (Nuclear Research Center, Beer-sheva, Israel) or 1 μ Ci [H$^3$] leucine were added to each well and the plate was incubated for 24—48 hours as above. The reaction was terminated as described in by Titerteck Cell Harvester precipitation, according to Hurwitz et al, ibid.

Antibody activity

The antibody activity of the platinum-Dex-NH$_2$-Ig complexes was determined by (a) Fluorescence analyzer cell sorter—tumor cells, 2×10$^6$ in 50 μl PBS containing 0,5% bovine serum albumin and 0,05% sodium azide were incubated at 0°C with 10—20 μg of antibody solutions in 50 μl for 30 min, washed 3 times and incubated for additional 30 min with fluorescent labeled antibodies directed against the first antibody. The cells were then washed, fixed with paraformaldehyde, and analysed.

(b) Radio-immunoassay-tumor cells were incubated with antibody solutions as is (a), washed and incubated again with $^{125}$I-second antibody solutions. After washing, the cells were counted in a gamma counter. Complexes of platinum were prepared with free Dex-NH$_2$, or with Dex-NH$_2$ bound to normal goat Ig (NIg), goat anti YAC Ig, goat anti 38C 13 Ig and anti-3LL Ig from hybridoma lines monoclonal antibodies), respectively. The conditions described above for the preparation of Pt-Dex-NH-Ig were optimal as judged by the highest extent of Dex-NH$_2$ conjugation and platinum complexing while retaining most of the antibody activity. The amount of platinum complexed to the Dex-NH$_2$-Ig as determined by either method described above was comparable and was found to range between 40—60 moles platinum per mole Ig.

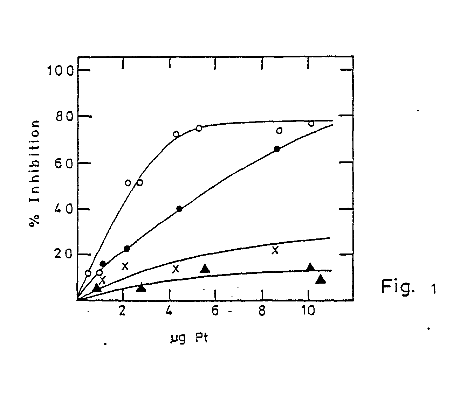

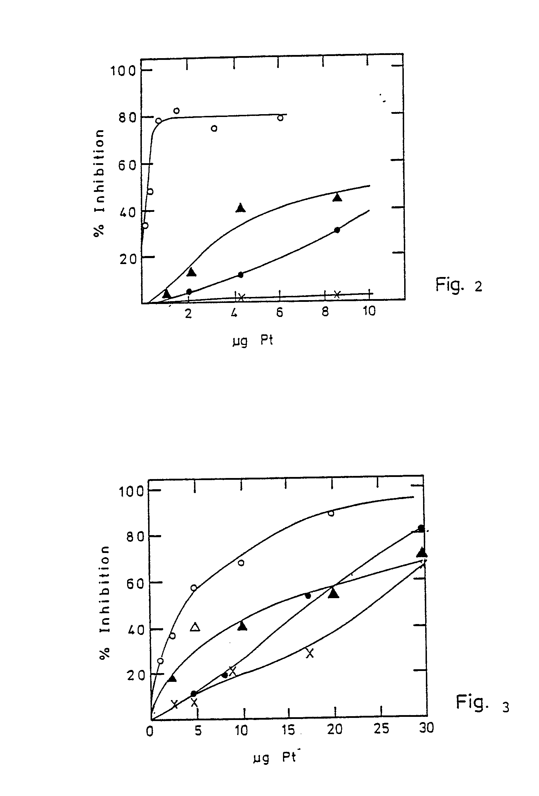

The pharmacological activity of the platinum conjugates was evaluated by inhibition of DNA synthesis, as measured by [$^3$H] methyl-thymidine incorporation. Figure 1 demonstrates the inhibition of DNA synthesis in YAC lymphoma cells. Pt-Dex-NH$_2$ anti-YAC Ig (Ig fraction of antiserum prepared against a soluble papain digest of YAC cell membrane) was a better inhibitor than K$_2$PtCl$_4$ (50% inhibition at 4,5 and 12 μg/ml respectively). All concentration values were calculated in platinum equivalents. As shown in Fig. 2, Pt-Dex-NH$_2$ and Pt-Dex-NH$_2$-NIg were both poor inhibitors. A more significant effect was observed in the system of the B leukemia (38C 13) cells (Fig. 2): Higher concentrations of K$_2$PtCl$_4$ were required for DNA inhibition (50% at 25—30 μg/ml). However, when complexed to Dex-NH$_2$ anti-38C Ig (idiotypic antibodies directed against the specific surface IgM of 38 cells), the platinum was a very effective inhibitor, leading to 50% inhibition at 0,7 μg/ml after 48 hrs of incubation (inhibition after 24 hrs was only slightly lower, with 50% inhibition at 1 μg/ml). Pt-Dex-NH$_2$-NIg was a much weaker inhibitor (50% at 20 μg/ml) and Pt-Dex-

$NH_2$ was a poor inhibitor. Platinum complexes with Dex-$NH_2$ derivatives of monoclonal antibodies directed against 3LL tumor cells were more effective than either free $K_2PtCl_4$ or the Pt complexes with Dex-$NH_2$-NIg or Dex-$NH_2$ (50% inhibition at 8, 33, 27 and 46 µg/ml, respectively) (Fig. 3).

The tumor cell binding capacity of the Pt-Dex-$NH_2$-Ig specific complexes was mostly retained as judged by the fluorescent cell sorter analyser experiments and by radioimmunoassays. Since both assays are indirect, at least some loss of antibody activity might be attributed not necessarily to loss of combining site function but also to decrease of secondary antibody binding of the modified first antibody.

The mechanism of the platinum anti-tumor activity is not known. In vitro it was shown to interact with DNA. Several in vivo studies suggested an involvement of the immune system in promoting platinum action. Immunodepressing compounds such as hydrocortisone were shown to decrease platinum potency, while immunostimulants such as zymosan were shown to increase it.

Preliminary experiments in vivo indicate that the novel complexes have a lower toxicity than that of the conventional cis-diaminoplatinum dihalides. Furthermore, the dosages required for an equal effect are lower than those of the conventional cis-DDp or of platinum complexes with various amides, nucleic acid bases and the like.

The platinum-antibody conjugates of the present invention are characterized by a therapeutic activity at least equal to that of the conventionally used cis-platinum compositions. The general toxicity of the conjugates of the present invention is lower, and thus for equal dosages, a lower overall toxicity will result. This makes possible the administration of larger dosages, if required, with a toxicity not exceeding that of the dosages used nowadays. Due to the specificity of the novel compositions, i.e. their recognition of the tumor cells due to the specific conjugated antibodies, the result is a "targeting" of the drug to the site of the tumor, reducing general systemic toxicity and increasing the local effectivity of the drug which appears in concentrated form at the very site where it is needed, i.e. at the site of the tumor where the cis-platinum exerts its full activity.

Fig. 1 illustrates the inhibition of [$^3$H] thymidine incorporation onto DNA of YAC cells ($5 \times 10^4$ cells/well) by Pt-Dex-$NH_2$anti YAC Ig (o), $K_2PtCl_4$ (o), Pt-Dex-$NH_2$ (x) and Pt-Dex-$NH_2$-NIg (Δ). Cells were incubated with the platinum compounds (or with PBS for control) for 21 hrs at 37°C after which [$^3$H] methylthymidine was added and incubated proceeded for 3 more hours;

Fig. 2 illustrates the inhibition of [$^3$H] thymidine incorporation of 38 leukemia cells (figure signs as in Fig. 1. Cells ($10^4$ cells/well) were incubated with the platinum compounds for 45 hrs (as above) followed by a 3 hrs pulse of [$^3$H] methylthymidine;

Fig. 3 illustrates the inhibition of [$^3$H] thymidine incorporation of 3LL cells ($5 \times 10^4$ cells/well) by Pt-Dex-$NH_2$ anti 3LL/hybridoma IgG) (o) $K_2PtCl_4$(o), Pt-Dex-$NH_2$(x) and Pt-Dex-NIg (Δ). Cells were incubated with the platinum compounds for 45 hrs followed by a 3 hrs pulse of [$^3$H] methylthymidine.

**Claims**

1. An anti-tumor agent wherein a platinum salt is complexed via a dextran-amine moiety to an anti-tumor immunoglobulin.

2. An anti-tumor agent according to claim 1, being the product of complexing of an immunoglobulin coupled to a dextran-amine with a platinum salt.

3. An anti-tumor agent according to claim 2, wherein the platinum salt is $K_2PtCl_4$.

4. An anti-tumor agent according to claim 1, wherein the immunoglobulin is specific for ovarian or testicular carcinoma or for renal adenocarcinoma.

5. An anti-tumor agent according to claim 1 in the form of an injectable liquid.

**Patentansprüche**

1. Anti-Tumormittel, worin ein Platinsalz über einen Dextran-amin-Anteil an ein Anti-Tumor-Immunoglobulin als Komplex gebunden ist.

2. Anti-Tumormittel nach Anspruch 1, das das Komplexierungsprodukt eines an ein Dextran-amin gekoppelten Immunoglobulins mit einem Platinsalz ist.

3. Anti-Tumormittel nach Anspruch 2, worin das Platinsalz $K_2PtCl_4$ ist.

4. Anti-Tumormittel nach Anspruch 1, worin das Immunoglobulin für ein ovariales oder testikulares Karzinom oder für ein renales Adenokarzinom spezifisch ist.

5. Anti-Tumormittel nach Anspruch 1 in Form einer injizierbaren Flüssigkeit.

**Revendications**

1. Un agent anti-tumeur dans lequel un sel de platine est complexé via une partie d'amine-dextrane à une immunoglobuline anti-tumeur.

2. Un agent anti-tumeur selon la revendication 1, étant le produit de complexation d'un immunoglobuline couplée à une amine-dextrane avec un sel de platine.

3. Un agent anti-tumeur selon la revendication 2, dans laquelle le sel de platine est $K_2PtCl_4$.

4. Un agent anti-tumeur selon la revendication 1, dans laquelle l'immunoglobuline est spécifique pour carcinome ovarien ou testiculaire ou pour l'adénocarcinome rénale.

5. Un agent anti-tumeur selon la revendication 1 sous la forme d'un liquide injectable.

Fig. 1

Fig. 2

Fig. 3